# FASCICULE DE BREVET EUROPEEN

(11) **EP 1 817 065 B1**
(45) Date de publication et mention de la délivrance du brevet: **23.02.2011**
(21) Numéro de dépôt: 05818937.4
(22) Date de dépôt: 28.11.2005
(51) Int. Cl.: A61L 31/04, A61L 24/04, A61L 24/08, A61K 9/00, A61K 47/36

(54) **SOLUTIONS VISCOELASTIQUES RENFERMANT DU HYALURONATE DE SODIUM ET DE L'HYDROXYPROPYLMETHYLCELLULOSE**
VISKOELASTISCHE LÖSUNGEN MIT NATRIUMHYALURONAT UND HYDROXYPROPYLMETHYLCELLULOSE, HERSTELLUNG UND VERWENDUNGEN
VISCOELASTIC SOLUTIONS CONTAINING SODIUM HYALURONATE AND HYDROXYPROPYLMETHYL- CELLULOSE, PREPARATION AND USES

(30) Priorité: 30.11.2004 FR 0412662
(43) Date de publication de la demande: 15.08.2007
(73) Titulaire: Corneal Innovation, 75002 Paris (FR)
(72) Inventeur: LEBRETON, Pierre, F-74940 Annecy le vieux (FR)
(74) Mandataire: Schwarz, Albin
(86) Numéro de dépôt international: PCT/FR2005/050996
(87) Numéro de publication internationale: WO 2006/059029

(56) Documents cités:
- WO-A-95/07085
- WO-A-96/32929
- WO-A-03/059391
- SILVER FREDERICK H ET AL.: "PHYSICAL PROPERTIES OF HYALURONIC ACID AND HYDROXYPROPYLMETHYLCELLULOSE IN SOLUTION; EVALUATION OF COATING ABILITY" JOURNAL OF APPLIED BIOMATERIALS, vol. 5, no. 1, 1994, pages 89-98, XP008050195 NY, USA
- LIESEGANG T J: "VISCOELASTIC SUBSTANCES IN OPHTHALMOLOGY" SURVEY OF OPHTHALMOLOGY, SURVEY OF OPHTHALMOLOGY INC, vol. 34, no. 4, 1990, pages 268-293, XP000650316 ISSN: 0039-6257

## Description

La présente invention a pour objet de nouvelles solutions viscoélastiques renfermant du hyaluronate de sodium (NaHA) et de l'hydroxypropylméthylcellulose (HPMC). Il s'agit de solutions aqueuses biocompatibles, convenant tout particulièrement à titres d'auxiliaires et/ou d'implants temporaires de chirurgie. La présente invention a également pour objet la préparation et l'utilisation desdites solutions comme auxiliaires et/ou implants temporaires de chirurgie et comme implants d'hydratation.

Toute invasion chirurgicale est dommageable pour les tissus. Pour minimiser les dommages en cause, notamment dans des zones où les tissus sont particulièrement fragiles et/ou irremplaçables, il est connu d'utiliser des solutions viscoélastiques comme auxiliaires de chirurgie. De telles solutions protègent les tissus des instruments chirurgicaux et aident à la manipulation desdits tissus. Elles sont également utilisées pour maintenir des espaces ou volumes, pour éviter des coalescences de tissus annihilant de tels espaces ou volumes. Des solutions de ce type sont tout particulièrement utilisées en chirurgie ophtalmique, plus spécifiquement en chirurgie de la cataracte.

En référence à ladite chirurgie de la cataracte, il a ainsi déjà été proposé les produits commerciaux ci-après :
- le Viscoat^{®}, par la société Alcon Surgical, Inc, qui renferme du hyaluronate de sodium (NaHA) et du sulfate de chondroïtine. Ce produit est aujourd'hui leader sur le marché ;
- les Healon^{®} et Healon GV^{®}, aujourd'hui commercialisés par la société A.M.O., les Amvisc^{®} et Amvisc Plus^{®}, aujourd'hui commercialisés par la société Bausch & Lomb, le Vitrax^{®}, aujourd'hui commercialisé par la société A.M.O. et les Viscornéal^{®} et Biocornéal^{®}, commercialisés par la Demanderesse, qui renferment du hyaluronate de sodium (NaHA) ;
- l'Orcolon^{®}, par la société Optical Radiation Corporation, qui renfermait un polyacrylamide, aujourd'hui non disponible ; et
- l'Occucoat^{®}, par la société Storz, qui renferme de l'hydroxypropylméthylcellulose (HPMC).

Chacun de ces produits commerciaux présente des avantages et des inconvénients. Ainsi, le Viscoat^{®} est très performant pour adhérer et protéger les tissus, notamment l'endothélium cornéen. Toutefois, en fin d'intervention, il est délicat de retirer ce produit de la chambre antérieure et pour l'insertion de lentilles intraoculaires, d'autres produits sont plus performants que lui.

Des produits du même type ont également été décrits :
- dans la demande WO-A-95 07085. Il est plus précisément décrit dans ce document des solutions ophtalmiques de mucopolysaccharide modifié, renfermant une fraction viscoélastique consistant en de l'acide hyaluronique substitué par des groupes acyle de 3 à 20 atomes de carbone, de l'acide hyaluronique (HA), de l'hydroxypropylméthylcellulose (HPMC) ou des mélanges de ces composés. Ladite fraction ne renferme pas de sulfate de chondroïtine. Des solutions HA (de faible ou forte masse moléculaire)/HPMC (de faible ou forte masse moléculaire) sont décrites, plus précisément des solutions de ce type renfermant 2 % en poids d'HPMC et 1 % en poids d'HA. Ces solutions ont été préparées et testées à l'échelle du laboratoire ;
- dans la demande WO-A-96 32929. Il est plus précisément décrit dans ce document des solutions renfermant une substance visqueuse ou viscoélastique, dans un véhicule aqueux à pH et osmolalité contrôlés. Ladite substance peut être choisie parmi l'acide hyaluronique ou l'un de ses sels et des mélanges acide hyaluronique ou l'un de ses sels/ cellulose modifiée ou collagène modifié. L'acide hyaluronique ou l'un de ses sels intervient à raison de 0,1 à 5 %, à une masse moléculaire comprise entre 0,5.10⁶ et 2,5.10⁶ ; la cellulose ou le collagène modifié à raison de 0,1 à 5 % également. Les solutions décrites ne sont pas réellement identifiées (aucune précision n'est fournie sur la masse moléculaire du collagène modifié ou de la cellulose modifiée, susceptible d'intervenir (l'inventeur a mis en évidence l'importance de la masse moléculaire de l'hydroxypropylméthylcellulose sur les qualités, notamment optiques, des solutions de l'invention (voir l'exemple 5 ci-après)) ... aucune information n'est donnée sur la réelle nature des composés associés dans les exemples 3 à 6 ... aucune information n'est donnée sur les mode de préparation et méthode de purification des produits) et elles n'ont visiblement été préparées et testées qu'à l'échelle du laboratoire.

Par ailleurs, WO-A-03 059391 décrit une méthode chirurgicale au cours de laquelle interviennent successivement :
- un agent viscoélastique à base d'un premier ingrédient (hyaluronate), puis
- une solution d'irrigation, très fluide, renfermant un deuxième ingrédient. Il ne propose pas, pour utilisation dans le cadre de ladite méthode chirurgicale, de solutions incorporant lesdits deux ingrédients.

Les mélanges testés dans les exemples, représentatifs de mélanges réalisés *in situ* (à l'intérieur de l'oeil) ne sont pas des solutions au sens de l'invention (la solution d'irrigation ne fait que modifier les propriétés de surface de l'agent viscoélastique). Les mélanges réalisés *in situ* ne sont pas des mélanges homogènes et par ailleurs, des microbulles sont *a priori* inexorablement générées à l'interface agent viscoélastique/solution d'irrigation.

L'enseignement de WO-A-03 059391 n'anticipe ni ne suggère l'objet de la présente invention (solutions, caractérisées par la nature de leurs constituants, leurs concentrations et masses moléculaires respectives (voir ci-après), prêtes à l'emploi). Au contraire, on peut objectivement considérer qu'il éloigne dudit objet l'homme du métier. Ledit enseignement, d'utiliser successivement deux ingrédients, est loin de suggérer la possibilité de préparer préalablement (industriellement) des solutions incorporant ces deux types d'ingrédients.

Le document *"*Physical Properties of Hyaluronic Acid and Hydroxypropylmethylcellulose in Solution: Evaluation of Coating Ability" de F. Silver et al. (Journal of Applied Biomaterials, Vol. 5, 89-98, 1994) décrit: des solutions aqueuses viscoélastiques biocompatibles à base de mélanges d'acide hyaluronique et d'hydroxypropylméthylcellulose ayant une faible masse moléculaire et étant utilisées en chirurgie ophtalmique.

Dans un tel contexte, il est proposé, selon l'invention, de nouvelles solutions viscoélastiques qui sont performantes, en terme de viscosité, d'élasticité, d'adhésion, d'étalement et de recouvrement des tissus ; et
qu'il est possible d'obtenir, présentant une grande qualité optique, dans des conditions industrielles intéressantes.

Les solutions de l'invention sont des solutions aqueuses viscoélastiques biocompatibles à base d'un mélange de hyaluronate(s) de sodium (NaHA) et d'hydroxypropylméthylcellulose(s) (HPMC). Elles renferment en fait au moins un hyaluronate de sodium (NaHA) d'une masse moléculaire moyenne donnée et au moins une hydroxypropylméthylcellulose (HPMC) d'une masse moléculaire moyenne donnée ; elles renferment généralement du hyaluronate de sodium (NaHA) d'une masse moléculaire moyenne donnée et de l'hydroxypropylméthylcellulose (HPMC) d'une masse moléculaire moyenne donnée.

De façon caractéristique, les solutions aqueuses viscoélastiques biocompatibles de l'invention renferment :
- de 1 à 2 % en poids, avantageusement de plus de 1 à 2% en poids, d'au moins un hyaluronate de sodium dont la masse moléculaire moyenne est comprise entre 1.10⁶ g/mol et 3,5.10⁶ g/mol ; et
- de 0,2 à 1 % en poids, avantageusement de 0,2 à moins de 1% en poids, d'au moins une hydroxypropylméthylcellulose dont la masse moléculaire moyenne est comprise entre 10 000 g/mol et 110 000 g/mol.

Le hyaluronate de sodium (NaHA) intervient principalement en référence aux propriétés rhéologiques tandis que l'hydroxypropylméthylcellulose (HPMC) intervient principalement en référence aux propriétés de surface.

De façon tout à fait inattendue, au sein des solutions de l'invention :
- ladite HPMC développe lesdites propriétés de surface, en intervenant à faible concentration (≤ 1 % en poids, avantageusement < 1% en poids) et à une faible masse moléculaire (≤ 110 000 g/mol). L'intervention d'une telle HPMC, de faible masse moléculaire, à faible concentration, facilite l'obtention des solutions de l'invention à l'échelle industrielle. Les problèmes de filtration et de qualité optique du produit (transparence, absence de bulles) restent, dans ces conditions, tout à fait gérables ;
- un effet de synergie est observé, avec une telle HPMC, sur la viscosité. Ceci est particulièrement intéressant dans un contexte d'utilisation des solutions pour maintenir des espaces ou volumes. En effet, plus le produit est visqueux et moins il est nécessaire d'en utiliser pour remplir un volume. Moins on en utilise et moins il est susceptible d'en rester dans l'oeil après l'intervention. L'homme du métier n'ignore pas les risques inhérents à la non-évacuation de la totalité du produit introduit dans l'oeil, risque d'élévation de la pression intraoculaire notamment.

Les variantes avantageuses ci-dessus peuvent être considérées indépendamment l'une de l'autre et, avantageusement, en combinaison l'une avec l'autre.

Les propriétés intéressantes des compositions de l'invention sont montrées dans les exemples ci-après.

De façon préférée, les solutions aqueuses viscoélastiques de l'invention renferment :
- de 1,2 à 1,8 % en poids d'au moins un hyaluronate de sodium dont la masse moléculaire moyenne est comprise entre 1,6. 10⁶ g/mol et 3.10⁶ g/mol ; et
- de 0,35 à 0,8 % en poids d'au moins une hydroxypropylméthylcellulose dont la masse moléculaire moyenne est comprise entre 10 000 g/mol et 50 000 g/mol.

De façon générale, et donc aussi dans le cadre de cette variante préférée, on associe avantageusement aux plus faibles concentrations les plus fortes masses moléculaires et vice-versa (on fait ainsi avantageusement intervenir un NaHA à 1,2 %, de masse moléculaire moyenne d'environ 3.10⁶ g/mol ou un NaHA à 1,8 %, de masse moléculaire moyenne d'environ 1,6.10⁶ g/mol).

On rappelle ici, à toutes fins utiles, que les solutions de l'invention sont tout à fait susceptibles de renfermer au moins deux hyaluronates de sodium, de masses moléculaires moyennes différentes (comprises entre 1.10⁶ g/mol et 3,5.10⁶ g/mol) et/ou au moins deux hydroxypropylméthylcelluloses, de masses moléculaires moyennes différentes (comprises entre 10 000 g/mol et 110 000 g/mol). Toutefois, elles renferment généralement un hyaluronate de sodium d'une masse moléculaire adéquate (comprise entre 1.10⁶ g/mol et 3,5.10⁶ g/mol, avantageusement entre 1,6.10⁶ g/mol et 3.10⁶ g/mol) et une hydroxypropylméthylcellulose d'une masse moléculaire adéquate (comprise entre 10 000 g/mol et 110 000 g/mol, avantageusement entre 10 000 g/mol et 50 000 g/mol).

Dans le cadre de variantes avantageuses, les solutions de l'invention renferment :
1,2 % en poids d'un hyaluronate de sodium dont la masse moléculaire moyenne est d'environ 3.10⁶ g/mol ; ou
1,37 % en poids d'au moins un hyaluronate de sodium dont la masse moléculaire moyenne est comprise entre 2.10⁶ et 3.1.0⁶ g/mol ; ou
1,8 % en poids d'un hyaluronate de sodium dont la masse moléculaire moyenne est d'environ 1,6.10⁶ g/mol.

Dans le cadre d'une autre variante avantageuse, à considérer indépendamment des ou en combinaison avec les variantes avantageuses ci-dessus, les solutions de l'invention renferment : 0,57 % en poids d'une hydroxypropylméthylcellulose dont la masse moléculaire moyenne est d'environ 20 000 g/mol.

Les solutions de l'invention, biocompatibles, sont avantageusement tamponnées à pH physiologique (pH = 7). Le tampon en cause est avantageusement un tampon phosphate.

Pour la préparation des solutions de l'invention, on préconise de procéder de la manière suivante :
- préparer du hyaluronate de sodium purifié, de masse(s) moléculaire(s) adéquate(s);
- préparer une solution purifiée adéquate (en référence à la concentration) d'hydroxypropylméthylcellulose(s), de masse(s) moléculaire(s) adéquate(s) ;
- dissoudre une quantité adéquate (en référence à la concentration) dudit hyaluronate de sodium purifié dans ladite solution ;
- homogénéiser la solution résultante ;
- débarrasser ladite solution résultante, des éventuels agglomérats qu'elle contient, par filtration.

Un tel procédé constitue le second objet de la présente invention.

Le hyaluronate de sodium (d'une masse moléculaire moyenne adéquate, comprise entre 1.10⁶ g/mol et 3,5.10⁶ g/mol ou d'au moins deux masses moléculaires moyennes adéquates, comprises entre 1.10⁶ g/mol et 3,5.10⁶ g/mol), intervient généralement à l'état de fibres tandis que l'hydroxypropylméthylcellulose (d'une masse moléculaire moyenne adéquate, comprise entre 10 000 g/mol et 110 000 g/mol ou d'au moins deux masses moléculaires moyennes adéquates, comprises entre 10 000 g/mol et 110 000 g/mol) intervient généralement à l'état de poudre.

Les purifications sont menées indépendamment l'une de l'autre dans le cadre du procédé de l'invention. On précise ci-après des variantes avantageuses de mise en oeuvre desdites purifications, particulièrement adaptées à la nature des produits en cause.

L'étape de filtration du mélange est avantageusement menée sur au moins un filtre de 5 µm.

Cette étape de filtration peut en tout état de cause être mise en oeuvre dans des conditions et délais raisonnables, au vu des faible concentration et masse moléculaire de l'(des) hydroxypropylméthylcellulose(s) présent(s) dans la solution.

Pour l'obtention du hyaluronate de sodium purifié, on préconise, au vu de la forte viscosité des solutions renfermant ce produit, de mettre en oeuvre la purification en amont. De façon avantageuse, on préconise de procéder comme suit.

On dissout des fibres de NaHA (de masse(s) moléculaire(s) adéquate(s)) à faible concentration. On filtre les solutions diluées obtenues sur des filtres de plus en plus fins. On préconise notamment de les filtrer successivement sur des filtres de 5 µm, 1 µm puis 0,22 µm.

Les solutions purifiées sont ensuite traitées de sorte qu'en leur sein, précipite le NaHA, purifié. La précipitation est généralement mise en oeuvre dans l'alcool. A l'issue d'un séchage, on récupère des fibres de NaHA purifié. L'homme du métier connaît ce processus de purification du hyaluronate de sodium.

Un processus de ce type est difficilement applicable à l'hydroxypropylméthylcellulose qui ne présente pas les mêmes caractéristiques de solubilité que le hyaluronate de sodium. Pour être précipité, il doit être chauffé ; ce qui, en milieu industriel, peut entraîner une prolifération bactérienne, source d'endotoxines.

De plus, ce processus de purification ne résoud en rien les phénomènes d'opalescence.

Pour la préparation de solutions purifiées d'hydroxypropylméthylcellulose (solutions précurseurs), on préconise, selon l'invention, de procéder par filtration. La matière première est dissoute, la solution obtenue est homogénéisée puis filtrée. Elle subit en fait des filtrations successives, sur des filtres de plus en plus fins. On rappelle ici que de telles solutions sont difficilement filtrables et que bien évidemment la difficulté augmente avec la concentration en hydroxypropylméthylcellulose.

On préconise de filtrer successivement sur des filtres de 5 µm, 1 µm puis 0,22 µm.

Ainsi, de manière particulièrement avantageuse, les solutions de l'invention sont obtenues, comme suit :
- la matière première (fibres) NaHA a été préalablement purifiée (par filtration d'une solution diluée de celle-ci, sur des filtres de 5 µm, 1 µm puis 0,22 µm) ;
- la matière première (poudre) HPMC a été dissoute dans une solution aqueuse adéquate. Ladite solution est successivement filtrée sur des filtres de 5 µm, 1 µm puis 0,22 µm ;
- le NaHA purifié est ajouté, en quantité adéquate, à la solution filtrée ; celle-ci est homogénéisé ;
- la solution homogénéisée est finalement filtrée sur un filtre de 5 µm.

Ce procédé de préparation des solutions de l'invention a notamment été mis en oeuvre pour la préparation des solutions des exemples ci-après.

On peut par ailleurs avantageusement améliorer la qualité de la solution obtenue en mettant en oeuvre sur celle-ci un dégazage. Un tel dégazage est destiné à débarrasser la solution viscoélastique obtenue des petites bulles de gaz, qu'elle est susceptible de renfermer.

La solution obtenue est ensuite généralement conditionnée puis stérilisée. Elle est généralement conditionnée dans des seringues.

On note incidemment ici que la stérilisation mise en oeuvre est susceptible de modifier quelque peu les masses moléculaires des NaHA et HPMC présents dans la solution.

Selon son troisième objet, la présente invention concerne l'utilisation des solutions de l'invention comme auxiliaires et/ou implants temporaires de chirurgie et comme implants d'hydratation.

On a parlé d'auxiliaires et/ou d'implants temporaires de chirurgie dans l'introduction du présent texte. Les solutions de l'invention sont particulièrement performantes dans ce contexte d'utilisation, sont donc particulièrement performantes dans un contexte de chirurgie de la cataracte. Dans un tel contexte d'utilisation, les solutions de l'invention sont injectées, remplissent leur fonction, puis récupérées en fin d'intervention.

Le débouché desdites solutions n'est pas limité à ce contexte. Elles conviennent également parfaitement comme implants d'hydratation, dans un contexte par exemple de méso-lifting. De tels implants d'hydratation sont injectés, remplissent leur fonction et disparaissent sur leur site d'injection. Ils y sont métabolisés.

Les solutions de l'invention sont généralement utilisées telles quelles mais il n'est pas exclu qu'il leur soit ajouté au moins un additif, qu'elles soient chargées en au moins un principe actif... Les implants (de chirurgie ou d'hydratation) et/ou auxiliaires mentionnés ci-dessus consistent donc ou consistent donc essentiellement en les solutions de l'invention.

On se propose maintenant d'illustrer l'invention et d'en mettre en avant l'intérêt par les exemples ci-après.

Les exemples 1, 2 et 3 montrent la synergie, annoncée ci-dessus sur la viscosité. La viscosité dynamique des gels testés a été mesurée avec un rhéomètre à contrainte imposée CARIMED CSL 500 (de TA Instruments), à une température de 25°C, avec un dispositif de mesure cône/plan (4 cm, 2°). La viscosité dynamique au repos est déterminée par une mesure à l'équilibre sous une contrainte de 1 Pa.

L'exemple 4 illustre les problèmes rencontrés à la filtration de solutions d'HPMC.

L'exemple 5 montre l'importance des paramètres : concentration et masse moléculaire de l'HPMC utilisé, sur la qualité optique de la solution.

### Exemple 1

On a préparé trois solutions aqueuses à partir des ingrédients ci-après :
- fibres de NaHA, de masse moléculaire Mw ≈ 2.5.10⁶ g/mol ;
- poudre de HPMC, commercialisée par DOW, connue sous la dénomination de Méthocel, référencée E4M, de masse moléculaire Mw ≈ 86.10³ g/mol.

La première solution, renfermant le NaHA, à une concentration de 1,28 % en poids, présentait une viscosité au repos de 234 ± 10 Pa.s.

La seconde, renfermant l'HPMC, à une concentration de 2 % en poids (en deçà, les solutions sont si peu visqueuses qu'il n'est pas possible de mesurer leur viscosité avec la méthode employée), présentait une viscosité au repos de 4 Pa.s.

La troisième solution, solution de l'invention, renfermant le même NaHA à la concentration de 1,28 % en poids et la même HPMC à la concentration de 0,32 %, présentait une viscosité au repos de 417 ± 12 Pa.s. On observe donc une variation de + 183 Pa.s alors que la HPMC ne contribue normalement que faiblement à la viscosité (voir le 4 Pa.s énoncé ci-dessus, pour une concentration environ 6 fois plus importante).

### Exemple 2

De la même façon, on a préparé trois solutions aqueuses à partir des ingrédients ci-après :
- fibres de NaHA, de masse moléculaire Mw = 3. 10⁶ g/mol ;
- poudre de HPMC, commercialisée par Dow, connue sous la dénomination de Méthocel, référencée E 50, de masse moléculaire Mw = 20.10³ g/mol.

La première solution, renfermant le NaHA, à une concentration de 1,21 % en poids, présentait une viscosité au repos de 213 ± 9 Pa.s.

La seconde, renfermant l'HPMC, à une concentration de 2 % en poids, présentait une viscosité au repos de 0,05 Pa.s.

La troisième solution, solution de l'invention, renfermant le même NaHA à la concentration 1,21 % en poids et la même HPMC à la concentration de 0,47 % en poids, présentait une viscosité au repos de 234 ± 8 Pa.s. L'augmentation de viscosité observée dans ce cas là reste supérieure à celle attendue (synergie) mais est plus mesurée que celle observée dans l'exemple précédent.

### Exemple 3

De la même façon, on a préparé trois solutions aqueuses à partir des ingrédients ci-après :
- fibres de NaHA, de masse moléculaire Mw ≈ 2,5.10⁶ g/mol ;
- poudre de HPMC, commercialisée par DOW, connue sous la dénomination de Méthocel, référencée E50, de masse moléculaire Mw ≈ 20 000 g/mol.

La première solution, renfermant le NaHA, à une concentration de 1,5 % en poids, présentait une viscosité au repos de 161 ± 2 Pa.s.

La seconde, renfermant le HPMC, à une concentration de 2 % en poids, présentait une viscosité au repos de 0,05 Pa.s.

La troisième solution, solution de l'invention, renfermant le même NaHA à la concentration de 1,5 % en poids et le même HPMC à la concentration de 0,57 %, présentait une viscosité au repos de 185 ± 7 Pa.s. L'effet de synergie est encore observé dans ce contexte.

### Exemple 4

Des solutions homogènes d'HPMC sont obtenues par dissolution de quantités adéquates de poudres (Méthocel commercialisé par DOW) dans des solutions de tampon phosphate.

L'homogénéisation est obtenue par agitation mécanique, à température ambiante. Après 48 h d'agitation, les solutions sont purifiées, par filtrations successives, jusqu'à filtration sur des filtres de 0,2 µm.

On a mesuré la quantité de solution filtrée en fonction du temps. Les résultats obtenus, avec des HPMCs, de masse moléculaire et/ou à des concentrations différentes, sont indiqués dans le tableau ci-après.

| HPMC | Concentration | Type filtration Qté/temps |
|---|---|---|
| HPMC 20 000 g/mol | 0,57 % | Filtre 0,2 µm 4 000 ml en 10 min |
| HPMC 20 000 g/mol | 2,0 % | Filtre 1,2 µm 115 ml en 20 min |
| HPMC 86 000 g/mol | 1,5 % | Filtre 1,2 µm 2 ml en 30 min |

Les chiffres ci-dessus confirment que plus la concentration en HPMC et/ou la masse moléculaire de HPMC est élevée, plus la filtration est difficile. L'intérêt économique de l'invention est évident.

On note incidemment que l'opération finale de filtration doit en fait être mise en oeuvre, dans le cadre de l'invention, sur des solutions renfermant en sus de l'HPMC, le NaHA.

### Exemple 5

On a préparé une solution de l'invention contenant :
1,2 % en poids de NaHA de Mw ≈ 3.10⁶ g/mol ; et
1 % en poids de HPMC de Mw = 110 000 g/mol.

La solution présente des phénomènes d'opalescence, encore tolérables, mais inéluctables.

Dans ces conditions limites de l'invention, en référence à l'HPMC (limites supérieures en concentration et en masse moléculaire), on n'obtient pas un produit de qualité optimale. On rappelle que ledit produit est généralement destiné à une utilisation en ophtalmologie...

On saisit ici tout l'intérêt des solutions de l'invention par rapport à celles de l'art antérieur décrites dans les documents WO-A-95 07085 et WO-A-96 32929, généralement plus concentrées en HPMC, éventuellement de plus forte masse moléculaire (préparées seulement à l'échelle du laboratoire).

## Revendications

1. Solution aqueuse viscoélastique biocompatible à base d'un mélange de hyaluronate(s) de sodium (NaHA) et d'hydroxypropylméthylcellulose(s) (HPMC), **caractérisée en ce qu'**elle renferme :
- de 1 à 2 % en poids, avantageusement de plus de 1 à 2 % en poids, d'au moins un hyaluronate de sodium dont la masse moléculaire moyenne est comprise entre 1.10⁶ g/mol et 3,5.10⁶ g/mol ; et
- de 0,2 à 1 % en poids, avantageusement de 0,2 à moins de 1% en poids, d'au moins une hydroxypropylméthylcellulose dont la masse moléculaire moyenne est comprise entre 10 000 g/mol et 110 000 g/mol.

2. Solution selon la revendication 1, **caractérisée en ce qu'**elle renferme :
- de 1,2 à 1,8 % en poids d'au moins un hyaluronate de sodium dont la masse moléculaire moyenne est comprise entre 1,6.10⁶ g/mol et 3.10⁶ g/mol ; et
- de 0,35 à 0,8 % en poids d'au moins une hydroxypropylméthylcellulose dont la masse moléculaire moyenne est comprise entre 10 000 g/mol et 50 000 g/mol.

3. Solution selon la revendication 1 ou 2, **caractérisée en ce qu'**elle renferme du hyaluronate de sodium d'une masse moléculaire adéquate et de l'hydroxypropylméthylcellulose d'une masse moléculaire adéquate.

4. Solution selon l'une quelconque des revendications 1 à 3, **caractérisée en ce qu'**elle renferme : 1,2 % en poids d'un hyaluronate de sodium dont la masse moléculaire moyenne est d'environ 3.10⁶ g/mol ; ou
1,37 % en poids d'au moins un hyaluronate de sodium dont la masse moléculaire moyenne est comprise entre 2.10⁶ et 3.10⁶ g/mol ; ou
1,8 % en poids d'un hyaluronate de sodium dont la masse moléculaire moyenne est d'environ 1,6.10⁶ g/mol.

5. Solution selon l'une quelconque des revendications 1 à 4, **caractérisée en ce qu'**elle renferme : 0,57 % en poids d'une hydroxypropylméthylcellulose dont la masse moléculaire moyenne est d'environ 20 000 g/mol.

6. Solution selon l'une quelconque des revendications 1 à 5, **caractérisée en ce qu'**elle est tamponnée à pH physiologique.

7. Solution selon la revendication 6, **caractérisée en ce qu'**elle est tamponnée par un tampon phosphate.

8. Procédé de préparation d'une solution selon l'une quelconque des revendications précédentes, **caractérisé en ce qu'**il comprend :
- la préparation de hyaluronate(s) de sodium purifié(s), de masse(s) moléculaire(s) moyenne(s) comprise entre 1.10⁶ g/mol et 3,5.10⁶ g/mol ;
- la préparation d'une solution purifiée d'hydroxypropylméthylcellulose(s) de masse(s) moléculaire(s) moyenne(s) comprise entre 10 000 g/mol et 110 000 g/mol ; ladite solution renfermant le(s)dit(s) hydroxypropyl-méthylcellulose(s) à une concentration permettant, après la mise en oeuvre de l'étape de dissolution ci-après, l'obtention de la concentration précisée ci-après ;
- la dissolution d'une quantité dudit(desdits) hyaluronate(s) de sodium purifié(s) pour l'obtention d'une solution renfermant de 1 à 2 % en poids, avantageusement de plus de 1 à 2 % en poids, d'au moins un hyaluronate de sodium purifié et de 0,2 % à 1 % en poids, avantageusement de 0,2 à 1 % en poids, d'au moins une hydroxypropylméthylcellulose ;
- l'homogénéisation de la solution résultante ; suivie de
- la filtration de ladite solution résultante.

9. Procédé selon la revendication 8, **caractérisé en ce que** ladite préparation de hyaluronate(s) de sodium purifié(s) comprend la mise en solution, à faible concentration, de hyaluronate(s) de sodium, des filtrations successives sur des filtres de plus en plus fins de la solution obtenue puis la précipitation dudit(desdits) hyaluronate(s) de sodium ainsi purifié(s).

10. Procédé selon la revendication 8 ou 9, **caractérisé en ce que** ladite préparation de la solution purifiée d'hydroxypropylméthylcellulose(s) comprend des filtrations successives sur des filtres de plus en plus fins.

11. Auxiliaires et/ou implants temporaires de chirurgie, **caractérisés en ce qu'**ils consistent en ou consistent essentiellement en une solution selon l'une quelconque des revendications 1 à 7 ou en une solution préparée selon l'une quelconque des revendications 8 à 10.

12. Implants d'hydratation, **caractérisés en ce qu'**ils consistent en ou consistent essentiellement en une solution selon l'une quelconque des revendications 1 à 7 ou en une solution préparée selon l'une quelconque des revendications 8 à 10.

## Claims

1. A biocompatible viscoelastic aqueous solution on the basis of a mixture of (a) sodium hyaluronate(s) (NaHA) and (a) hydroxypropyl methylcellulose(s), **characterised in that** it comprises:
- 1 to 2 % by weight, advantageously more than 1 to 2 % by weight, of at least one sodium hyaluronate having an average molecular weight between 1x10⁶ g/mol and 3.5x0⁶ g/mol; and
- 0.2 to 1 % by weight, advantageously 0.2 to less than 1 % by weight, of at least one hydroxypropyl methylcellulose having an average molecular weight between 10,000 g/mol and 110,000 g/mol.

2. The solution according to claim 1, **characterised in that** it comprises:
- 1.2 to 1.8 % by weight of at least one sodium hyaluronate having an average molecular weight between 1x6 10⁶ g/mol and 3x 10⁶ g/mol; and
- 0.35 to 0.8 % by weight of at least one hydroxypropyl methylcellulose having an average molecular weight between 10,000 g/mol and 50,000 g/mol.

3. The solution according to claim 1 or claim 2, **characterised in that** it comprises sodium hyaluronate having an adequate molecular weight and hydroxypropyl methylcellulose having an adequate molecular weight.

4. The solution according to any one of the claims 1 to 3, **characterised in that** it comprises:
- 1.2 % by weight of a sodium hyaluronate having an average molecular weight of about 3x10⁶ g/mol; or
- 1.37 % by weight of a sodium hyaluronate having an average molecular weight between 2x 10⁶ and 3x 10⁶ g/mol; or
- 1.8 % by weight of a sodium hyaluronate having an average molecular weight of about 1. 6x 10⁶ g/mol.

5. The solution according to any one of the claims 1 to 4, **characterised in that** it comprises: 0.57% by weight of a hydroxypropyl methylcellulose having an average molecular weight of about 20,000 g/mol.

6. The solution according to any one of the claims 1 to 5, **characterised in that** it is buffered at a physiological pH.

7. The solution according to claim 6, **characterised in that** it is buffered with a phosphate buffer.

8. A procedure for preparing the solution according to any one of the preceding claims, **characterised in that** it comprises:
- preparing (a) purified sodium hyaluronate(s) having an average molecular weight between 1x10⁶ g/mol and 3.5x10⁶ g/mol;
- preparing a purified solution of (a) hydroxypropyl methylcellulose(s) having an average molecular weight between 10,000 g/mol and 110,000 g/mol; said solution comprising said hydroxypropyl methylcellulose(s) in a concentration which makes it possible to obtain the concentrations specified below after carrying out the dissolution step described below;
- dissolving a quantity of said purified sodium hyaluronate(s) in order to obtain a solution comprising 1 to 2 % by weight, advantageously more than 1 to 2 % by weight, of at least one purified sodium hyaluronate and 0.2 to 1 % by weight, advantageously 0.2 to 1 % by weight, of at least one hydroxypropyl methylcellulose;
- homogenising the resulting solution; followed by
- filtrating said resulting solution.

9. The procedure according to claim 8, **characterised in that** said preparation of the purified sodium hyaluronate(s) comprises the dissolution of the sodium hyaluronate(s) at a low concentration, consecutive filtrations of the obtained solution through increasingly fine filters, and the precipitation of the thus purified sodium hyaluronate(s).

10. The procedure according to claim 8 or claim 9, **characterised in that** said preparation of the purified solution of hydroxypropyl methylcellulose(s) comprises consecutive filtrations through increasingly fine filters.

11. Auxiliaries and/or temporary surgical implants, **characterised in that** they consist or essentially consist of a solution according to any one of the claims 1 to 7, or a solution prepared according to any one of the claims 8 to 10.

12. Hydrating implants, **characterised in that** they consist or essentially consist of the solution according to any one of the claims 1 to 7, or a solution prepared according to any one of the claims 8 to 10.

## Patentansprüche

1. Bioverträgliche viskoelastische wässrige Lösung auf Basis eines Gemischs von Natriumhyaluronat(en) (NaHA) und Hydroxypropylmethylcellulose(n) (HPMC), **dadurch gekennzeichnet, dass** sie Folgendes umfasst:
- 1 bis 2 Gew.-%, vorteilhafterweise mehr als 1 bis 2 Gew.-%, zumindest eines Natriumhyaluronats, dessen mittleres Molekulargewicht zwischen 1x10⁶ g/mol und 3,5x10⁶ g/mol liegt; und
- 0,2 bis 1 Gew.-%, vorteilhafterweise 0,2 bis weniger als 1 Gew.-%, zumindest einer Hydroxypropylmethylcellulose, deren mittleres Molekulargewicht zwischen 10.000 g/mol und 110.000 g/mol liegt.

2. Lösung nach Anspruch 1, **dadurch gekennzeichnet, dass** sie Folgendes umfasst:
- 1,2 bis 1,8 Gew.-% zumindest eines Natriumhyaluronats, dessen mittleres Molekulargewicht zwischen 1,6x10⁶ g/mol und 3x10⁶ g/mol liegt; und
- 0,35 bis 0,8 Gew.-% zumindest einer Hydroxypropylmethylcellulose, deren mittleres Molekulargewicht zwischen 10.000 g/mol und 50.000 g/mol liegt.

3. Lösung nach Anspruch 1 oder 2, **dadurch gekennzeichnet, dass** sie Natriumhyaluronat mit geeignetem Molekulargewicht und Hydroxypropylmethylcellulose mit geeignetem Molekulargewicht umfasst.

4. Lösung nach einem der Ansprüche 1 bis 3, **dadurch gekennzeichnet, dass** sie Folgendes umfasst:
- 1,2 Gew.-% eines Natriumhyaluronats, dessen mittleres Molekulargewicht etwa 3x 10⁶ g/mol beträgt; oder
- 1,37 Gew.-% eines Natriumhyaluronats, dessen mittleres Molekulargewicht zwischen 2x 10⁶ und 3x 10⁶ g/mol liegt; oder
- 1,8 Gew.-% eines Natriumhyaluronats, dessen mittleres Molekulargewicht etwa 1,6x 10⁶ g/mol beträgt.

5. Lösung nach einem der Ansprüche 1 bis 4, **dadurch gekennzeichnet, dass** sie Folgendes umfasst: 0,57 Gew.-% einer Hydroxypropylmethylcellulose, deren mittleres Molekulargewicht etwa 20.000 g/mol beträgt.

6. Lösung nach einem der Ansprüche 1 bis 5, **dadurch gekennzeichnet, dass** sie auf einen physiologischen pH gepuffert ist.

7. Lösung nach Anspruch 6, **dadurch gekennzeichnet, dass** sie durch einen Phosphatpuffer gepuffert ist.

8. Verfahren zur Herstellung einer Lösung nach einem der vorangegangenen Ansprüche, **dadurch gekennzeichnet, dass** es Folgendes umfasst:
- die Herstellung von (einem) gereinigten Natriumhyaluronat(en) mit einem mittleren Molekulargewicht zwischen 1 x 10⁶ g/mol und 3,5x 10⁶ g/mol;
- die Herstellung einer gereinigten Lösung von (einer) Hydroxypropylcellulose(n) mit einem mittleren Molekulargewicht zwischen 10.000 g/mol und 110.000 g/mol; wobei die Lösung die Hydroxypropylcellulose(n) in einer Konzentration umfasst, die nach Durchführung des nachstehenden Lösungsschritts den Erhalt der nachstehend angeführten Konzentration ermöglicht;
- das Lösen einer Menge des besagten gereinigten Natriumhyaluronats/der besagten gereinigten Natriumhyaluronate zum Erhalt einer Lösung, die 1 bis 2 Gew.-%, vorteilhafterweise mehr als 1 bis 2 Gew.-%, zumindest eines gereinigten Natriumhyaluronats und 0,2 bis 1 Gew.-%, vorteilhafterweise 0,2 bis 1 Gew.-%, zumindest einer Hydroxypropylmethylcellulose umfasst;
- das Homogenisieren der resultierenden Lösung; gefolgt von
- der Filtration besagter resultierenden Lösung.

9. Verfahren nach Anspruch 8, **dadurch gekennzeichnet, dass** besagte Herstellung des gereinigten Natriumhyaluronats/der gereinigten Natriumhyaluronate das Lösen des Natriumhyaluronats/der Natriumhyaluronate in geringer Konzentration, aufeinander folgende Filtrationen der erhaltenen Lösung durch immer feinere Filter und anschließend die Ausfällung besagten, auf diese Weise gereinigten Natriumhyaluronats/besagter, auf diese Weise gereinigten Natriumhyaluronate umfasst.

10. Verfahren nach Anspruch 8 oder 9, **dadurch gekennzeichnet, dass** die Herstellung besagter gereinigten Lösung von Hydroxypropylmethylcellulose(n) aufeinander folgende Filtrationen durch immer feinere Filter umfasst.

11. Hilfsvorrichtungen und/oder temporäre chirurgische Implantate, die **dadurch gekennzeichnet sind, dass** sie aus einer Lösung nach einem der Ansprüche 1 bis 7 oder einer Lösung, die gemäß einem der Ansprüche 8 bis 10 hergestellt ist, bestehen oder im Wesentlichen aus einer Lösung nach einem der Ansprüche 1 bis 7 oder einer Lösung, die gemäß einem der Ansprüche 8 bis 10 hergestellt ist, bestehen.

12. Feuchtigkeitsversorgungsimplantate, die **dadurch gekennzeichnet sind, dass** sie aus einer Lösung nach einem der Ansprüche 1 bis 7 oder einer Lösung, die gemäß einem der Ansprüche 8 bis 10 hergestellt ist, bestehen oder im Wesentlichen aus einer Lösung nach einem der Ansprüche 1 bis 7 oder einer Lösung, die gemäß einem der Ansprüche 8 bis 10 hergestellt ist, bestehen.
